# EUROPEAN PATENT APPLICATION

(11) **EP 1 747 715 A1**
(43) Date of publication of application: **31.01.2007**
(21) Application number: 04738255.1
(22) Date of filing: 18.06.2004
(51) Int. Cl.: A01H 1/00, A01H 1/02

(54) **THE SELECTION METHODS OF SELF-POLLINATION AND NORMAL CROSS POLLINATION IN POPULATION, VARIETY OF CROPS**

(30) Priority: 26.04.2004 CN 200410034154; 26.04.2004 CN 200410034155; 26.04.2004 CN 200410034156
(71) Applicant: The Rice Research Insitute of Guangdong Academy of Agricultural Sciences, Tianhe Guangzhou Guangdong 510640 (CN); Yangtze University, Jingzhou Hubei 434025 (CN); Xiaofang, Li, Tianhe Guangzhou Guangdong 510640 (CN)
(72) Inventor: LI, Xiaofang Guangdong Academy of Agric. Sciences, Guangzhou Guangdong 510640 (CN); LU, Yanzhuo Guangdong Academy of Agric. Sciences, Guangzhou Guangdong 510640 (CN); XIAO, Xin Guangdong Academy of Agric. Sciences, Guangzhou Guangdong 510640 (CN); LUO, Wenyong Guangdong Academy of Agric. Sciences, Guangzhou Guangdong 510640 (CN); CHEN, Jianwei Guangdong Academy of Agric. Sciences, Guangzhou Guangdong 510640 (CN); MAO, Xingxue Guangdong Academy of Agric. Sciences, Guangzhou Guangdong 510640 (CN); WANG, Xiaoling Guangdong Academy of Agric.Sciences, Guangzhou Guangdong 510640 (CN); XING, Danying Guangdong Academy of Agric. Sciences, Guangzhou Guangdong 510640 (CN); ZHOU, Guiyuan Guangdong Academy of Agric. Sciences, Guangzhou Guangdong 510640 (CN)
(74) Representative: Schüssler, Andrea
(86) International application number: PCT/CN2004/000657
(87) International publication number: WO 2005/102032

(57) **Abstract**

The present invention relates to breeding of crop plants. It is specifically suitable for breeding of colony varieties or cultivars of self-pollinating and frequent-cross-pollinating plants. A female group is hybridized with a male group to produce colony varieties. The female group is produced as follows: Pairs of parental plants with different desired characteristics are hybridized to produce F1, and pairs of F1 are then hybridized to produce a segregation population. The individual plants in this segregation population or their self-crossed descendants in the early segregation generations will constitute the female group. The male group consists of homozygous breeding lines, varieties, or heterozygous plants in the segregation generations or individual plants that are produced in a similar manner to the production of the female group. The process of breeding colony varieties includes 5 phases: hybridization, self-crossing for stabilization, characterization, grouping assortment and formation of a colony. The colony varieties also possess features of the conventional varieties---uniformity, stability and peculiarity. Farmers can reserve their seeds for the next planting, and seed companies may explore further development based on stock seeds. The colony varieties have good adaptability, high yield and strong resistance to pests and diseases. Colony variety breeding has less reliance on breeders' experience and thus even farmers can do the breeding. In addition, 11 cases for breeding colony varieties in various crop plants and their performance in the production are cited in detail for demonstration.

## Description

### FIELD OF INVENTION

The present invention relates to the breeding of crop plants. It is specifically suitable for breeding of colony varieties or cultivars of self-pollinating plants (e.g. rice, peanut, wheat, soybean and tomato, etc) and frequent-cross-pollinating plants (e.g. rape, cotton, chili pepper, flowering Chinese cabbage, eggplant and cabbage mustard, etc).

### BACKGROUND OF INVENTION

Self-pollinating crop plants include rice, peanut, wheat, soybean and tomato, and frequent-cross-pollinating plants include rape, cotton, chili pepper, flowering Chinese cabbage, eggplant and cabbage mustard, etc. Among them, rice is the staple food for one third of world's population, and the production of cereals including rice is always an important issue for global economy. At present, there are two types of rice which are utilized in the production: conventional varieties and hybrid combinations. Both of them belong to a single genotype category. Conventional rice is a stable single-genotype variety which was developed after multi-generation selections of descendants of a cross or crosses between proper parental lines. The semi-dwarfication of rice is one of the breakthroughs in the history of rice breeding. G. S. Khush et al., from the International Research Institute (IRRI) successfully developed a semi-dwarf rice variety of IR8 in 1965, and Yaoxiang Huang et al., from the Rice Research Institute of Guangdong Academy of Agricultural Sciences of China (RRI, GAAS) developed a semi-dwarf rice variety of Guang-Chang-Ai in late 1950s. Both Khush and Huang made these important and influential contributions to rice breeding research worldwide(Huang, International rice Congress, 2002.9). Hybrid rice was the first successful case of a self-pollinating crop plant for heterosis utilization. Longping Yuan, an academician from China Hybrid Rice Research Centre, was a pioneer in the development of hybrid rice, and he was dubbed as 'the father of hybrid rice' (Yuan et al, International rice Congress 2002.9). China generally takes a leading position in rice breeding and production. Due to the prolonged efforts of a great many breeders, rice breeding technology has improved dramatically, and thousands of varieties including hybrid and conventional rice have been developed. However, one common feature of these varieties is single genotype, which means that all plants of a variety or a combination share one genotype.

For wheat, Norman Borloug developed a multi-line variety to control rust disease in 1959. He first hybridized 15 varieties, which were resistant separately to 11 different strains of pathogenic bacteria of rust, with Yaqui 50, an elite variety in agronomical traits; he then made a backcross hybridization using Yaquis 50 as the recurrent parental line. He finally developed an isogenic line containing 15 breeding lines, and mixed eight of them to form a multi-line variety. Derong Gao et al., also developed a multi-line variety of wheat which contained various genes for powdery mildew resistance (Gao et al, Anhui Agricultural Science, 2001, 29(5): 603-604). However, this wheat variety was not suitable for production development due to big variations in plant height among its isogenic lines. Zhaolin Fu believed that multi-line varieties would exhibit superiority in yield potential and stability, but generation of multi-line varieties would be related to methods and techniques of mixing of multiple lines. He thus raised the concept of ecological combining capacity and stressed uniformity in agronomical traits. He also mentioned the possibility of different responses of various genotypes to the environment. However, his considerations mainly focused on the mixture of 2-3 developed breeding lines within a multi-line variety. Liren Wu et al., (Agricultural Sciences in China, 2000, 33(5): 1-7) also developed multi-line varieties to control strip rust disease in wheat. The introgression of various resistance genes into the same variety was accomplished by them through repeated backcrosses. A number of isogenic lines with similar agronomical traits but with different resistance genes were developed and they were grown in a mixed-planting fashion according to their resistance level to strip rust. However, these lines also failed to be released for production due to difficultites in overcoming 'complexity and multiple purpose' of the breeding. Nevertheless, planting of mixed varieties is still of interest to many researchers. Yongmei Guo et al (Journal of Yunnan Agricultural University, 2001, 4: 267-270) brought forward the strategy of developing multi-line varieties for restorer lines of hybrid rice resistant to rice blast. The first step in the strategy is to hybridize different varieties, which are resistant to various strains of pathogenic fungi of blast, with elite varieties (restorer lines herein). The next step is to backcross the hybrids with restorer lines (recurrent parental lines) repeatedly to produce isogenic lines with varied resistance genes to blast. The final step is to manually mix a number of isogenic lines to form the multi-line varieties. The mixed population is known as the host group, which has the same genetic background except for varied resistance genes to blast, and the variability of the blast-resistance genes facilitates diversification of disease resistance. Thus, there is no doubt that multi-line varieties have superiority in disease resistance. Almost all of the present multi-line vareities have been applied in disease control. However, only simple hybridization, backcross and breeding selection were used in the development of these multi-line varieties. Tremendous work of backcross is needed if many multi-line varieties are to be developed. At present, the uniformity of multi-line varieties is still a big problem because the adopted strategy of 'best from the best', which is suitable to single-genotype variety breeding, would result in peculiarity of each breeding line. Therefore, the above-mentioned breeding of multi-line varieties only focuses on a specific major agronomical trait to obtain a multi-loci variety of that gene. Due to limitations of the 'best from the best' strategy, creation and selection of a great many recombinants are not involved in the above-mentioned breeding process. Difficulties in overcoming the "complexity and multi-purpose" of the breeding process have prevented commercial application of these breeding methods. Although mixed varieties have intrigued researchers, a great number of varieties with similar agronomical traits must be sought before mixing. Otherwise, uniformity of the varieties cannot be realized due to high specificity of each variety. In addition, infringement of intellectual property rights of each variety would also be a concern. Up to now, multi-genotype varieties in rice or wheat that are suitable for commercial production have not been actually developed.

Therefore, a new strategy is needed to develope multi-genotype colony varieties, which are characterized by uniformity, stability and specificity in phenotype. Dinstinct from the strategy of the aforementioned multi-line varieties for just disease resistance, the present invention can be applied in many areas of crop improvement.

### DETAILED DESCRIPTION OF INVENTION

This invention provides a method for breeding colony varieties or cultivars of crop plants which are characterized by uniformity, stability and specificity in phenotype, which provides improvements in quality, yield and resistance to disease and pests.

A female group is hybridized with a male group to produce the primary population of a given colony variety. The production of the female group follows as below. Pairs of parental plants with different predermined characteristics are hybridized to produce F1, and pairs of F1 are then hybridized one more time to produce the segregation population. The individual plants in this segregation population, or their self-crossed descendants in the early segregation generations, will constitute the female group. The male group can be homozygous breeding lines, varieties, F1, or heterozygous plants in the segregation generations or individual plants produced in the same manner as for the production of the female group. The female group may have 2-4 original parental lines. The parental lines may be homozygous breeding lines or varieties, or plants in the segregation generation which can be the descendants of single, double or triple crosses. If required by the breeding goal, the parental lines of single, double and triple crosses themselves could be the plants in the segregation generation of single, double or triple crosses. The individual plants in the segregation generation will be self-crossed for 1-10 generations. The number of individual plants for crossing with the male population is within the range between 4 and 10,000. Preferably, the range is between 6 and 10,000, and the most preferred range is between 1 and 5,000. The plants in the segregation generation of the male groups are the descendants of single, double or triple crosses.

Genetic Diversity Breeding: The number of parental lines for hybridization is more than six. Nearly all descendants of the hybridization will be retained and be self-crossed for stability to produce thousands of homozygous genotypes so that the genetic diversity of the plants will be maximally exploited. The stable individual plants are then divided into groups according to selection criteria and the characteristics of the desired varieties. Finally those groups of individual plants become various colony varieties. Since the genetic diversity is derived from numerous varieties and recombinations of hybridization which can be actively utilized in breeding, this strategy of breeding is called "genetic diversity breeding". This strategy of breeding may also be applied to the breeding of self-pollinating and frequent-cross-pollinating crop plants and therefore can be widely applied in the future.

Colony improvement: the above-mentioned homozygous multi-genotype population is known as the primary colony. This new breeding strategy mainly involves improvements in the multi-genotype colony for many times. The selection strategy is also different from that of the 'best from the best' as in conventional breeding. It focuses on selecting harmonic populations for the breeding goal, usually deleting the poorest but not selecting the best, so that the quality, yield and resistance to diseases and pests of the colony can be improved. Since the major object of this improvement process is a colony, it is also known as colony breeding.

Group or colony variety (multi-genotype colony variety): since this breeding strategy results in a colony of plants with multiple different genotypes rather than a group of single genotype plants resulted from current breeding strategy, it is called multi-genotype colony breeding. However, this colony varity consists of multiple genotype varieties having similar phenotype. For clarity and simplicity, a more common and short term for multi-genotype colony variety, colony variety or group variety, is adopted herein.

The principles of genetic diversity breeding are based on the fact that most of the agronomical traits of crop plants are quantitive traits which are quantitatively controlled by numerous delicate-effect genes. In another word, these delicate-effect genes interact each other among them and exert similar effects on the quantitive traits. Recent molecular biological research has identified the quantitative trait loci (QTLs) for many agronomical traits, which confirm this principle. Based on this principle, a multi-gene-controlled trait may show a seriese of identical or similar phenotypes but is essentially controlled by multiple genes. The essence of diversity genetic breeding is based on this principle, through alternative application of multiple gene controlled homogenous phenotype. In conventional and hybrid breeding, experts seek to breed toward a homogenic and single-genotype variety using techniques that are time-consuming and labor intensive. In our new breeding method, we have adopted a different strategy. In contrast, we just select those individual plants with identical or similar phenotypes, and retain as many recombinants with different genotypes as possible. Thus, it is unnecessary to concern about the differences in genotype among similar phenotypes. This new strategy is different from that of multi-line varieties for controlling blast disease of rice. The number of parental lines for hybridization of multi-line varieties is small, the homogenic lines were selected for them, and they were planted alternately in the field. The sole objective of their breeding was just to control blast disease of rice. No innovation was accomplished in the breeding strategy, such as hybridization, selection, retaintion of genetic diversity, classification of individual plants and colony improvement.

The detailed scheme for this invention is described below:

### 1. Hybridzation Phase:

The parental lines for hybridization of genetic diversity breeding should have some characteristics towards the breeding goal or provide some special characteristics. They can be domestic or foreign varieties so that their adaptability is increased. The characteristics of the parental lines should complement collectively, but it is unnecessary for pairs of parental lines to complement each other. Dozens of parental lines could be used for hybridization. At this phase, the most important thing is to create as many recombinants as possible at a cost of a minimum number of hybridizations.

The female group could be homogenic breeding lines, varieties or plants in the segregation generation of single, double or triple crosses of the original parental lines. The male group could also be homogenic breeding lines, varieties or plants in the segregation generation of single, double or triple crosses of the original parental lines.

In order to increase its impact on the colony variety, a certain variety could be used for hybridization more than one time as the parental line of the female or the male group depending on the situation. For instance, the female group could be (A×B) or (A×B) ×(A×C), the male group could be (E×F)×(F×H) and so on. If an inadequate amount of seeds were collected after hybridization of the parental lines of the female group, they could be self-crossed for 1-10 generations depending on the multiplication capacity of various crops. In other words, the female group could be plants in the segregation generation of the crosses. The number of individual plants in segregation for hybridization with the male group should be between 4 and 10,000, with 6-10,000 being a better range and 10-5,000 being the best range. As segregation mainly focuses on production of heterogenic plants, it is sufficient if adequate recombinants are produced for the female group.

As described above, when the female and male groups are produced, the workload for hybridization is not heavy since their parental lines are non-segregation ones and only moderate quantities of seeds are needed. Hybridizations between the female and male groups are more important. As each individual plant in the female or male group is different in genotype, the hybridization must be big enough to acquire adequate recombinants. Supplementary hybridization could also be made in later segregation generations. Although the segregation generations of crosses among more than 3 parental lines can be adopted for the production of male and female groups, it would increase hybridization circles and lengthen breeding duration. Thus it is preferred that only one cross is made between the female and male groups to complete the whole hybridization and production of the primary colony.

### 2. Self-crossing phase for stability:

After the hybridization is completed and the primary colony produced, the self-crossing phase will begin to achieve stability of the colony. The number of individual plants to be retained in each generation will be decided based on the multiplication capacity of the crops and the breeding goals. Each of the individual plant lines will be planted separately and multiplied by selfing until they reach stability in the phenotype. If an adequate quantity of seeds is produced for the primary colony, the seeds of only a small number of plants will be maintained for each individual plant line and they will be mixed together as the representatives in the early generations. If the primary colony produces only a small quantity of seeds, then the number of plants to be retained in early segregation generations for each individual plant line should be big enough so that as many genotypes are maintained as possible. The minimum number of individual plant lines for selfmg should be more than 5000. In the late generations, one plant will be retained for each individual plant line until full stability is nearly completed. Stability will be judged by the variation within an individual plant line. Some of the poorest plants will be eliminated from a line. However, the whole of the original individual lines in the primary colony should not be deleted. The workload will not be great for self-pollinating crops in this phase, since bagged selfing is unnecessary. The selfing process of colony breeding itself is no different than that of conventional breeding, but it has different purposes. For conventional breeding, the 'best from the best' plants will be selected in this phase until stable single-genotype plants are developed. For the present invention, on the contrary, the genetic diversity or multiple genotypes will be maintained as far as possible in this phase and only the poorest plants will be eliminated. In addition, shuttle breeding in different ecological areas could be conducted so that various genotypes would be fully expressed and their adaptability and coordinating ability would be assessed.

### 3. Characteristic measurement phase:

In this phase, some major characteristics of the stable recombinant individuals will be measured according to the breeding goals. Some basic characteristics, such as plant height and growth duration, should be included. This is a vast and repetitive task, which can be performed by responsible labour workers. If resources allow, quality and other characters, which are hard to measure, should be determined as well. To reduce cost, some major characteristics could be determined after the individual plants have been grouped in the colony. Sufficient seeds should be collected from each plant for subsequent planting and for conducting other determinations.

### 4. Grouping assortment phase

In order to be released for large scale development, a variety must first meet the requirement with uniformity of phenotype. The genotype of individual plants has become stable through selection in previous stages, but the phenotype may still be variable. The genotypes may be different although their phenotypes may be equal. The individual plant lines with equal phenotype (allowing for a limited variation) will be merged into a group. Their differences in genotype will not be taken for strict consideration but will be fully utilized. Taking rice as an example, if the difference in heading date is limited to plus or minus one day, the colony variety will become uniform in heading although the genotypes are different within it. The requirements for selection should be strict for appearance characteristics, such as growth duration and plant height, and it could be somewhat relaxed for other characters. The whole process of selection can be completed on computer, and multi-genotype colony varieties will be formed. These colony varieties will be uniform in characteristics if strict selection criteria are used, or they will exhibit certain differences in characters without strict selection critiera. The colony varieties will also show all-around advantages in quality, adaptability, yield and resistance to diseases and pests in spite of their inferiority to conventional varieties in uniformity of phenotype. If the cost for measurement is reduced with the development of this technology, more characteristics can be determined or measured to reach a finer assortment so that the uniformity of colony varieties can be further improved. However, the uniformity will have conflicts with the diversity of genotypes, an optimal balance should be attained between them.

After the completion of determination, the data will be processed by computer. The mean, maxium, minimum and mode values of colony varieties in some major characteristics will be calculated for selection and assortment. In order to avoid blindness and improve efficiency, the following principles should be considered.

Principle of Yield: after all individual plants are sorted by yield order, 20% of them with the lowest yield will be eliminated. If the yield is not considered to be a limiting factor, the percentage could be lowered to 10% e.g.

Principle of breeding expectation: This principle is exemplified using rice. If the expected growth duration to heading is 70 days and the expected plant height is 90cm, then plants with corresponding values of 69-71 days and 89-91 cm will be selected.

Principle of mode value of a current population: In practice, the selection strategy can be modified according to the real characteristics of the data of a population. For instance, the mode value of a characteristic can be adopted so that more genetic diversity could be contained in the population. For a population of rice with mode and expected values of 73 and 70 days respectively in heading, the genotypes within the expectation range (69-71 days) in heading may have only 50 plants, but the genotypes within the mode range (71-74 days) may have as many as 200 plants or even more. In such a case, the mode value will provide a higher probability and will enlarge the scope for selection in other characteristics.

Principle of mean value of a current population: selection can be made according to the mean value of a current population. For rice, if the mean plant height is 95cm, plants with a plant height of 94-96cm should be selected.

Principle of varied requirements for major and secondary characteristics: for instance, plant height and growth duration greatly affect the overall uniformity of a population, and there are relatively few genes controlling plant height. So the variation range for these characteristics should be narrow. In contrast, yield and maturity will be influenced greatly by enviromental conditions, a wide variation range for these characteristics could be allowed, allowing for even a plus or minus of 3%.

Principle of intercross selection: In practice, intercross selection can be made. For instance, a population could first be selected based on the mean values of heading and plant height, and then be selected again according to the mode of maturity or its highest yield, or around the modes of some major growth duration, and plants with expected plant height would be chosen. In our experience, a number of selection plans could be elaborately designed.

Principle of subcenter selection: If the values around the mean or mode are the center, the limits within the allowed fluctuation ranges of the mean or mode are then regarded as the subcenter. Those upper limits of the mean or mode value are the upper subcenter and those lower limits of the mean or mode are the lower subcenter. Selection around the subcenters will enlarge the scope of genetic diversity. Many recombinations of selection plans can be made by utilizing intercross selection around the upper and lower subceneters. Given no overlap in recombination and variation scope, a number of colony varieties can be developed from a population and maxium genetic diversity would be exploited. For instance, we once designed a plan of genetic diversity breeding using a group of 12 parental lines of rice. We have developed 20 colony varieties for one ecological area and 40 for two ecological areas.

Selection for peculiar purposes: according to a special breeding purpose, a particular colony variety can be developed beyond the center and subcenter in a specific characteristic. For rice, we once developed a colony variety with a late maturity of up to 130 days in growth duration, which could be particularily useful if planted in the single-cropping area in the Hubei province in China.

### 5. Formation and evaluation phase of colony variety

After completion of selection on computer, the seeds of the corresponding individual plants in the same group are mixed in equal ratios to form a colony variety with an uniform phenotype. The colony varieties are then planted and evaluated for their comprehensive performance in yield, pest and disease resistance and other characteristics. Some proper modification and amendment to the colony will also be made for flaws occurring in previous phases, such as incorrect scoring by the computer due to mistakes in measurement, aberrant plants caused by instability and obviously abnormal plants, which will impair the yield, quality, pest and disease resistance of the colony. Shuttle breeding can also be conducted to further optimize the colony and enhance its adaptability. Thereby, those processes can be repeated to further improve the colony for several generations. It should be noted that the focus here is not the selection of individual plants in the 'best from the best' fashion of conventional breeding. The overall coordination within the colony is more important for a durable impact on yield, quality and resistance to pests and diseases.

This invention can be specifically applied to common self-pollinating crops and frequent-cross-pollinating crops as well, such as rice, peanut, wheat, cotton, soybean, tomato, rape, chili pepper, flowering Chinese cabbage, eggplant, and cabbage mustard etc. They are enumerated below as non-limiting implemented cases.

### EMBODIMENTS OF THE INVENTION

The following examples are included to demonstrate preferred embodiments of the invention. It should be appreciated by those of skilled in the art that the techniques disclosed in the examples which follow represent techniques discovered by the inventors to function well in the practice of the invention, and thus can be considered to constitute preferred modes for its practice. However, those of skilled in the art should, in light of the present disclosure, appreciate that many changes can be made in the specific embodiments which are disclosed and still obtain a like or similar result without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### Example 1---Rice

### 1. Hybridization design

A) Experimental materials:
   8 parental lines of different origins were adopted in the experiment: Li-Xian-Zhan, Shan-Tai-Zhan 8, Wei-You 35, II-You 46, D-You 64, Wei-You 64, Shan-You 63 and Shan-You 64.
B) Experimental methods:
   (1) 2 original parental lines for the female group: Li-Xian-Zhan, Shan-Tai-Zhan 8
      Hybridizaton:
      Li-Xian-Zhan×Shan-Tai-Zhan 8: The produced descendents denoted as F₁ were self-crossed, and 10 individual plants in its segregation generation formed the female group;
      Wei-You 35×II-You 46, D-You 64×Wei-You 64, Shan-You 63×Shan-You 64: 1000 filial individual plants of the hybrids formed the male group and they were hybridized with the female group.
   (2) 3 original parental lines for the female group: Li-Xian-Zhan 8, Shan-Tai-Zhan 8, Wei-You 35
      Hybridization:
      (Li-Xian-Zhan 8×Shan-Tai-Zhan 8)×Wei-You 35: 500 individual plants in the segregation generation formed the female group;
      [(Wei-You 35×II-You 46)×(D-You 64×Wei-You 64)]: 100 individual plants in the segregation generation formed the male group;

### 2. Breeding of colony varieties, cultivars of rice:

Twelve parental plants with different origins were used in the experiment. Zhong-Er-Ruan-Zhan, Yue-Hua-Zhan, Yue-Tai-Zhan and Er-Ba-Zhan are high quality grain varieties developed by the Rice Research Institute of Guangdong Academy of Agricultural Science (RRI of GAAS) in China; Feng-Ai-Zhan and Yue-Xiang-Zhan are high-yielding varieties developed by RRI of GAAS; Qi-Gui-Zao and Te-Xian-Zhan 13 are high-yielding varieties developed by the Foshan Agricultural Research Institute in the Guangdong province of China; Duo-Kang 578, Duo-Kang 580 and Duo-Kang 583 are disease-resistant varieties introduced from the International Rice Research Institute in the Philippines; and Lemont is a famous U.S. high quality grain variety. Yue-Xiang-Zhan, the control in the experiment, has been used as a control variety for the national geographical rice trials and in rice trials in Guangdong province up to now.
A) Experimental methods:
(1)Piled-up hybridization
   The 1st hybridization: including the following six separate crosses:
   Feng-Ai-Zhan×Duo-Kang 578; Lemont×Zhong-Er-Ruan-Zhan;
   Yue-Tai- ZhanxDuo-Kang 580; Yue-Hua-Zhan×Er-Ba-Zhan;
   Te-Xian-Zhan 13×Yue-Xiang-Zhan; Qi-Gui-Zao×Duo-Kang 583

   The 2nd hybridization: involving simple crosses between the male and female of the F1 generation. As the first and secondary hybridizations were performed in non-segregation generations, the work was not great, and acquiring only dozens of seeds was needed. The crosses are listed below:
   (Feng-Ai-ZhanxDuo-Kang 578)×(Lemont×Zhong-Er-Ruan-Zhan);
   (Yue-Tai-Zhan×Duo-Kang 580)×(Yue-Hua-Zhan×Er-Ba-Zhan);
   (Te-Xian-Zhan13×Yue-Xiang-Zhan)×(Qi-Gui-Zao×Duo-Kang 583);

   The tertiary hybridization:
   [(Feng-Ai-ZhanxDuo-Kang 578) × (Lemont×Er-Ruan-Zhan)]---female × {[(Yue-Tai-ZhanxDuo-Kang 580) × (Yue-Hua-Zhan×Er-Ba-Zhan)] --- male 1+[(Te Xian Zhan 13×Yue Xiang Zhan) ×(Qi Gui Zao×Duo Kang 583)]---male 2}.

   Note: in the above notations, the female is placed in front and the male in back.
   First, each individual plant of the female population was numbered and so was that of the two male populations. The plants in the female population were then fertilized with the mixed pollens of plants in the two male populations, both of which had the same plant numbers of the corresponding female plants. In total, 214 hybrids have been produced.
(2) Self-cross of progenies for stabilization and investigation design:
   For the progenies of the 214 hybrids, at least 10 self-crossed plants were retained for each individual plant line, and they were self-crossed for 6 generations towards their stability. When they finally became stable, all individual plant lines were investigated for their heading and maturity dates and plant height at both heading and maturity stages. According to their mean and mode values and breeding purposes, the individual plants within the allowed variation in those values were chosen and merged into the same group through computer. A total of 20 colony varieties were developed and were designated as JT1, JT2...JT20. As they could be grown 2 times a year in Guangdong province, all of them have become 13^{th} generation by now.

B) Results of the experimental application:
For the progenies of the above hybrids, at least 10 self-crossed plants were retained for each individual plant line, and they were self-crossed for 6 generations towards their stability. When they finally became stable, all individual plant lines were investigated for their heading and maturity dates and plant height at both heading and maturity stages. According to their mean and mode values and the breeding purposes, the individual plants within the allowed variation in those values were chosen and merged into the same group through computer. A total of 20 colony varieties were developed and were designated as JT1, JT2...JT20. As they could be grown 2 times a year in Guangdong province, all of them have become 13th generation by now.
Characterization of colony varieties of rice: through screening and sorting, 20 colony varieties were developed according to the dates of heading and maturity and plant height at heading and maturity of individual lines of the colony. Some of their characteristics are shown in the Table 1. Amylose content is a major limiting factor for the grain quality of conventional varieties of Indica rice. The amylose contents of most current varieties goes beyond the range (16-23%) of the national grade 2 standard for quality rice in China, and only a smaller number of varieties can meet at the grade 1 standard (17-22%) in amylose content. Only around 5% of the varieties released up to now can reach a grade 1 or grade 2 standards for amylose content. In the breeding period, even fewer breeding lines met at the above standards for amylose content. 14 colony varieties, accounting for 85%, arrived at grade 2 or higher, and 5 varieties (accounting for 25%) arrived at grade 1. In comparison to conventional breeding methods, this new strategy greatly increased the probability of obtaining high-quality varieties that reached the national standard in amylose content. As a matter of fact, the quality of colony rice resembles that of formulated rice. This will benefit the farmers and enhance the efficiency of quality breeding of rice by more than 60%.

**Table 1: Characterization of colony varieties of rice**

| Code | Days to heading (d) | Plant height at heading (cm) | Days to maturity (d) | Plant height at maturity (cm) | Yield equivalent of first-level colony (Kg/1333 m²) | Yield equivalent of second-level colony (Kg/1333 m²) | Amylose content (%) |
|---|---|---|---|---|---|---|---|
| JT1 | 88.65854 | 65.4878 | 115.5854 | 91.46341 | 698.41 | 688.94 | 24.94 |
| JT2 | 91.68421 | 66.89474 | 119.4211 | 107.8947 | 756.13 | 781.49 | 24.86 |
| JT3 | 97.36364 | 70.90909 | 127.2727 | 116.8182 | 672.64 | 687.50 | 24.86 |
| JT4 | 100.1111 | 70.22222 | 126.8889 | 117.8889 | 842.49 | 863.84 | 25.56 |
| JT5 | 84.23077 | 63.15385 | 110.3846 | 101.9231 | 890.00 | 893.99 | **22.42** |
| JT6 | 84.66667 | 60.46667 | 108.7333 | 95.53333 | 848.88 | 822.96 | **20.00** |
| JT7 | 89.27273 | 63.72727 | 116.6364 | 82.90909 | 842.34 | 848.48 | 26.77 |
| JT8 | 87.13158 | 64.94737 | 115.2368 | 105.0789 | 888.88 | 893.61 | **22.21** |
| JT9 | 89.69231 | 65 | 116.0513 | 105.1538 | 902.49 | **921.05** | **21.89** |
| JT10 | 90.78125 | 65.125 | 119.125 | 104.875 | 939.53 | **952.38** | **22.99** |
| JT11 | 87.95 | 70.25 | 115.5 | 110.75 | 817.77 | 823.82 | 23.66 |
| JT12 | 90.69565 | 70.69565 | 117.3043 | 110.8261 | 743.76 | 757.73 | **22.89** |
| JT13 | 91.3 | 70.4 | 119.65 | 110.9 | 825.39 | 839.50 | 23.18 |
| JT14 | 90.15385 | 67.46154 | 116.7692 | 113.2308 | 933.33 | 937.79 | **22.71** |
| JT15 | 90.73333 | 69.53333 | 116.8667 | 113.5333 | 928.88 | **961.53** | **22.61** |
| JT16 | 92.13333 | 67.4 | 120.1333 | 113.3333 | 907.02 | 911.76 | **19.51** |
| JT17 | 91.1875 | 71 | 120.125 | 113.4375 | 879.81 | 898.98 | **19.91** |
| JT18 | 92.05882 | 66.70588 | 120.1765 | 115 | 973.95 | **974.52** | **22.09** |
| JT19 | 95.83333 | 70.58333 | 122.25 | 115 | 804.08 | 821.10 | **21.04** |
| JT20 | 91.92308 | 70.61538 | 120.0769 | 115 | 802.91 | 765.30 | **22.31** |
| average | 90.87808 | 67.52896 | 118.2094 | 108.0275 | 844.9345 | 852.3135 | **22.8205** |
| CK | 69.12 | 62.05 | 125.45 | 89.56 | | 918.08 | 26.78 |

### Example 2---Peanut

### 1. Hybridization design

A) Experimental materials:
   6 parental lines of different origins were adopted in the experiment: Pu-Hua 6, Hua-Xuan 1, Sui-Hua 6, Shan-You 321, Yue-You 79 and FU91-103
B) Experimental methods:
   (1) 2 original parental lines for the female group: Pu-Hua 6, Hua-Xuan 1
      Hybridizaton:
      Pu-Hua 6×Hua-Xuan 1: 10 individual plants in its F₂ segregation generation formed the female group;
      Xu-Hua 6×Shan-You 321, Yue-You 79×FU91-103: 1000 filial individual plants of the hybrids formed the male group and they were hybridized with the female group.
   (2) 3 original parental lines for the female group: Shan-You 321, Yue-You 79 and FU91-103
      Hybridization:
      (Shan-You 321×Yue-You 79)×FU91-103: 2000 individual plants in the segregation generation formed the female group;
      [(Hua-Xuan 1×Xu-Hua 6)×(Shan-You 321×Yue-You 79)]: 100 individual plants in the segregation generation formed the male group.

### 2. Breeding of colony varieties, cultivars of peanut:

A) Materials of the experiment
Intensified breeding of high-quality and comprehensive-character-chosen varieties: Below are the parental lines of crosses. 5 high-quality varieties including Zhong-Yue-You 223, Yue-You 202-35, Yue-You 39 (these three were developed by Guangdong Academy of Agricultural Sciences), Ah7223 (introduced from the USA) and Indian Hua-Pi (introduced from India); 4 high-yielding varieties including Yue-You 116, Yue-You 7 (developed by Guangdong Academy of Agricultural Sciences), Zhong-Hua 4 (developed by Oil Plants Research Institute, Chinese Academy of Agricultural Sciences), 8506-A (developed by Henan Academy of Agricultural Sciences); 3 disease-resistant varieties including Yue-You 200 (tolerant to bacterial wilt), Yue-You 79 (tolerant to bacterial wilt and rust) and Shan-You 523 (developed by Shantou Agricultural Research Institute). Hybridization was conducted between pairs of the above varieties.
B) Experimental methods
The 1 st hybridization:
Yue-You 223×Yue-You 79; 8506-A×Yue-You 200; Yue-You 200-35×Yue-You 116; Zhong-Hua 4×Indian Hua-Pi; Yue-You 39×Yue-You 7; Ah7223×Shan-You 523;

The 2nd hybridization
(Yue-You 223×Yue-You 79) F₁× (8506-A×Yue-You 200) F₁, 50 seeds collected; (Yue-You 200-35×Yue-You 116) F₁ ×(Zhong-Hua 4×Indian Hua-Pi) F₁; (Yue-You 39×Yue-You 7) F₁ ×( Ah7223×Shan-You 523) F₁

The tertiary hybridization:
[(Yue-You 223×Yue-You 79) F₁× (8506-A×Yue-You 200) F₁] F₁×{[(Yue-You 200-35×Yue-You 116) F₁ ×(Zhong-Hua 4×Indian Hua-Pi) F₁] F1---male 1+ [(Yue-You 39×Yue-You 7) F₁ ×(Ah7223×Shan-You 523) F₁]---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 220 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine rouging could be performed until it became stable and uniform. In total, three colony varieties of JTHS1, JTHS2 and JTHS3 were developed, and their growth duration and plant height are listed below:

| Code | Growth duration (d) | Plant height (cm) |
|---|---|---|
| JTHS1 | 130 | 58 |
| JTHS2 | 120 | 45 |
| JTHS3 | 110 | 51 |

C) Results of the experimental application
Through screening and sorting, 20 colony varieties were developed according to the dates of flowering and maturity and plant height at pegging and maturity of individual lines of the colony. Some of their characteristics are shown in Table 2. Low yield is a major limiting factor for the conventional varieties of peanut, and protein content is lower than 32% for most peanut varieties, with only 5% of varieties having a protein content of more than 32%. In the breeding period, even fewer breeding lines have a protein content of higher than 32%. In this experiment, 14 colony varieties, accounting for 70% of the total, had protein contents higher than 32% and 1 variety (accounting for 5%) had a protein content of higher than 34%. In comparison to conventional breeding methods, this new strategy greatly increased the probability of obtaining high-quality varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As they belong to a self-pollinating plant, their stability would remain steady during multiplication.

**Table 2 Characterization of colony varieties of peanut**

| Code | Protein content (%) | Yield (Kg/667m²) | Yield increase over control (%) | Plant height (cm) | Growth duration to maturity (d) |
|---|---|---|---|---|---|
| JTP11 | 32.57 | 356 | 37.45174 | 110 | 130 |
| JTP12 | 32.56 | 344 | 32.81853 | 108 | 130 |
| JTP1 | 34.01 | 332 | 28.18533 | 86 | 130 |
| JTP13 | 32.21 | 332 | 28.18533 | 94 | 130 |
| JTP2 | 33.83 | 320 | 23.55212 | 102 | 130 |
| JTP14 | 32.03 | 320 | 23.55212 | 84 | 128 |
| JTP3 | 33.64 | 308 | 18.91892 | 79 | 128 |
| JTP 15 | 31.74 | 308 | 18.91892 | 74 | 128 |
| JTP4 | 33.47 | 296 | 14.28571 | 69 | 128 |
| JTP16 | 31.47 | 296 | 14.28571 | 64 | 125 |
| JTP5 | 33.3 | 284 | 9.65251 | 59 | 125 |
| JTP17 | 31.21 | 284 | 9.65251 | 86 | 125 |
| JTP6 | 33.11 | 272 | 5.019305 | 74 | 125 |
| JTP18 | 30.94 | 272 | 5.019305 | 64 | 125 |
| JTP7 | 32.95 | 260 | 0.3861 | 55 | 115 |
| JTP19 | 29.68 | 260 | 0.3861 | 46 | 120 |
| JTP8 | 32.93 | 248 | -4.2471 | 49 | 120 |
| JTP20 | 28.41 | 248 | -4.2471 | 52 | 120 |
| JTP9 | 32.85 | 236 | -8.88031 | 55 | 120 |
| JTP10 | 32.67 | 224 | -13.5135 | 58 | 120 |

### Example 3---Wheat

### 1. Hybridization design

A) Experimental materials
   6 parental lines of different origins were adopted in the experiment: Shaan-Nong 757, Tai T15, JI-Shen-Cang 6001, Jin-Mai 54, Ke-Nong 9204, Yu-Mai 46.
B) Experimental methods
   (1) 2 original parental lines of the female group: Shaan-Nong 757, Tai T15
      Hybridization: Shaan-Nong 757×Tai T15, 10 plants in the F₂ segregation generation formed the female group; JI-Shen-Cang 6001×Jin-Mai 54, Ke-Nong 9204×Yu-Mai 46, 1000 individual plants of hybrids formed the male group and were hybridized separately with the female group.
   (2) 3 original parental lines of the female group: Jin-Mai 54, Ke-Nong 9204, Yu-Mai 46.
      Hybridization: (Jin-Mai 54×Ke-Nong 9204)×Yu-Mai 46, 2000 plants in the segregation generation formed the female group; [(Tai T15×JI-Shen-Cang 6001) ×(Jin-Mai 54×Ke-Nong 9204)], 100 plants in the segregation generation formed the male group.

### 2. Breeding of colony varieties, cultivars of wheat

A) Materials of experiment
Intensified breeding of high-quality and comprehensive-character-chosen varieties: Below are the parental lines of crosses. Liao-Chun 12 and Shen-Mian 96 (from Liaoning), Ken-Jiu 10 and Ken-Hong 14 (from Heilongjiang), Chi-Mai 5 and Meng-Mai 30 (from inner Mongolia), Yang-Mai 10 (from Jiangsu), Gan-Chun 20 (from Gansu), Quan-Mai 3 (from Fujian), Yan-Zhan 4110 (from Henan), Chuan-Yu 5404 and Yu-Mai 7 (from Chongqin). Hybridization was conducted between pairs of the above varieties.
B) Experimental methods
The first hybridization:
Liao-Chun 13×Yang-Mai 10; Shen-Mian 96×Gan-Chun 20; Ken-Jiu 10×Quan-Mai 3; Ken-Hong 14×Yan-Zhan 4110; Chi-Mai 5×Chuan-Yu 5404; Meng-Mai 30×Yu-Mai 7.

The secondary hybridization:
(Liao-Chun 13×Yang-Mai 10) F₁×(Shen-Mian 96xGan-Chun 20) F₁, 50 seeds were collected.
(Ken-Jiu 10xQuan-Mai 3) F₁×(Ken-Hong 14×Yan-Zhan 4110) F₁, 50 seeds were collected.
(Chi-Mai 5×Chuan-Yu 5404) F₁×(Meng-Mai 30xYu-Mai 7) F₁, 50 seeds were collected.

The tertiary hybridization:
[(Liao-Chun 13×Yang-Mai 10) F₁×(Shen-Mian 96×Gan-Chun 20) F₁] ×{[(Ken-Jiu 10×Quan-Mai 3) F₁×( Ken-Hong 14×Yan-Zhan 4110) F₁] --- male 1+ [(Chi-Mai 5×Chuan-Yu 5404) F₁×(Meng-Mai 30×Yu-Mai 7) F₁]---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 220 hybrids were obtained from 50 female plants. One self-crossed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JTXM1, JTXM2 and JTXM3 were developed, and their growth duration and plant height are listed below:

| Code | Growth duration to maturity (d) | Plant height (cm) |
|---|---|---|
| JTXM1 | 101 | 95 |
| JTXM2 | 91 | 87 |
| JTXM3 | 104 | 98 |

C) Results of the experimental application

Through screening and sorting, 16 colony varieties were developed according to the dates of flowering and maturity and plant height of individual lines of the colony. Some of their characteristics are shown in Table 3. Low rough protein content is a major limiting factor for the conventional varieties of wheat, and protein content is lower than 17% for most wheat varieties, with only a minority of varieties having a protein content of higher than 17%. In the breeding period, even fewer breeding lines had a protein content of higher than 17%. In this experiment, 8 colony varieties, accounting for 50% of the total, had a protein content of higher than 17% and 1 variety had protein content of higher than 22%. In comparison to conventional breeding methods, this new strategy greatly increased the probability of obtaining high-quality varieties. The uniformity of the colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As they belong to a self-pollinating plant, their stability would remain steady during multiplication.

**Table 3 Characterization of colony varieties of wheat**

| Code | Rough protein content (%) | Increase over control (%) | Plant height (cm) | Growth duration to maturity (cm) |
|---|---|---|---|---|
| JTXM1 | 15.56 | -1.44 | 110 | 114 |
| JTXM2 | 16.44 | -0.54 | 108 | 118 |
| JTXM3 | 16.02 | -0.98 | 86 | 120 |
| JTXM4 | 17.23 | +0.23 | 94 | 120 |
| JTXM5 | 16.90 | -0.1 | 102 | 88 |
| JTXM6 | 18.07 | +1.07 | 84 | 108 |
| JTXM7 | 19.64 | +2.64 | 79 | 105 |
| JTXM8 | 15.74 | -1.26 | 74 | 89 |
| JTXM9 | 16.47 | -0.53 | 77 | 98 |
| JTXM10 | 20.47 | +3.47 | 94 | 101 |
| JTXM11 | 19.44 | +2.44 | 99 | 98 |
| JTXM12 | 16.78 | -0.12 | 74 | 120 |
| JTXM13 | 22.75 | +5.75 | 86 | 89 |
| JTXM14 | 19.97 | +2.97 | 89 | 95 |
| JTXM15 | 15.85 | -1.15 | 85 | 79 |
| JTXM16 | 19.67 | +2.67 | 98 | 88 |

### Example 4--Soybean

### 1. Hybridization design

A) Experimental materials
   Six parental lines of different origins were adopted in the experiment: Ken-Nong 18, Ji-Yu 47, Liao-Dou 1, He-Feng 41, Ji-Yu 58, Liao-Dou 13.
B) Experimental methods
   (1) 2 original parental lines of the female group: Ken-Nong 18, Ji-Yu 47
      Hybridization: Ken-Nong 18×Ji-Yu 47, 10 plants in the F₂ segregation generation formed the female group; Liao-Dou 1×He-Feng 41, Ji-Yu 58×Liao-Dou 13, 1000 individual plants of hybrids formed the male group, and they were hybridized separately with the female group.
   (2) 3 original parental lines of the female group: He-Feng 41, Ji-Yu 58, Liao-Dou 13.
      Hybridization: (He-Feng 41×Ji-Yu 58)×Liao-Dou 13, 2000 plants in the segregation generation formed the female group; [(Ji-Yu 47×Liao-Dou 1) ×(He-Feng 41×Ji-Yu 58)], 100 plants in the segregation generation formed the male group.

### 2. Breeding of colony varieties, cultivars of soybean

A) Materials of the experiment
Intensified breeding of high-quality and comprehensive-character-chosen varieties: Below are the parental lines of crosses. Ji-Yu 35 and Ji-lin 20 (from Liaoning), Hei-Nong 46 and Feng-Shou 24 (from Heilongjiang), Cang-Dou 5 and Wu-Xing 1(from Hebei), Yu-Dou 29 and Zhen 90007 (from Henan), Xu-Dou 12 (from Jiangsu), Jin-Yi 30 (from Shanxi), Qi-Huang (from Shandong), He-Dou 3 (from Anhui). Hybridization was conducted between pairs of the above varieties.
B) Experimental methods
The first hybridization:
Ji-Yu 35×Jin-Yi 30; Zhen 90007×Xu-Dou 12; JI-Lin 20×Cang-Dou 5; Yu-Dou 29×He-Dou 3; He-Nong 46×Wu-Xing 1; Feng-Shou 24×Qi-Huang 29.

The secondary hybridization:
(Ji-Yu 35×Jin-Yi 30) F₁×(Zhen 90007×Xu-Dou 12) F₁, 50 seeds were collected.
(JI-Lin 20×Cang-Dou 5) F₁×(Yu-Dou 29×He-Dou 3) F₁, 50 seeds were collected.
(He-Nong 46×Wu-Xing 1) F₁×(Feng-Shou 24×Qi-Huang 29) F₁, 50 seeds were collected.

The tertiary hybridization:
[(Ji-Yu 35×Jin-Yi 30) F₁×(Zhen 90007×Xu-Dou 12) F₁] F₁×{[(JI-Lin 20×Cang-Dou 5) F₁×(Yu-Dou 29×He-Dou 3) F₁] F₁---male 1+ [(He-Nong 46×Wu-Xing 1) F₁×(Feng-Shou 24×Qi-Huang 29)F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 220 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Growth duration to maturity (d) | Plant height (cm) |
|---|---|---|
| JT1 | 125 | 97 |
| JT2 | 108 | 78 |
| JT3 | 135 | 105 |

C) Results of the experimental application
Through screening and sorting, 12 colony varieties were developed according to the podding habits, growth duration and plant height of individual lines of the colony. Some of their characteristics are shown in Table 4. Low rough fat content is a major limiting factor for conventional varieties of soybean, and the fat content is between 20%-21% for most soybean varieties. In the breeding period, even fewer breeding lines have a fat content of higher than 22%. In this experiment, 7 colony varieties, accounting for 58% of the total, had a fat content of higher than 22% and the highest one had a fat content of up to 24%. In comparison to conventional breeding methods, this new strategy greatly increased the probability of obtaining high-quality varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As they belong to a self-pollinating plant, their stability would remain steady during multiplication.

**Table 4 Characterization of colony varieties of soybean**

| Code Code | Rough fat content (%) | Increase over control (%) | Plant height (cm) | Growth duration (d) | Podding habit |
|---|---|---|---|---|---|
| JTDD1 | 22.54 | 37.45174 | 110 | 130 | sub-finite |
| JTDD2 | 22.67 | 32.81853 | 108 | 130 | sub-finite |
| JTDD3 | 24.01 | 28.18533 | 86 | 130 | sub-finite |
| JTDD4 | 21.21 | 28.18533 | 94 | 130 | sub-finite |
| JTDD5 | 19.83 | 23.55212 | 102 | 130 | finite |
| JTDD6 | 23.03 | 23.55212 | 84 | 128 | finite |
| JTDD7 | 20.64 | 18.91892 | 79 | 128 | finite |
| JTDD8 | 21.74 | 18.91892 | 74 | 128 | sub-finite |
| JTDD9 | 23.47 | 14.28571 | 69 | 128 | sub-finite |
| JTDD10 | 21.47 | 14.28571 | 64 | 125 | sub-finite |
| JTDD11 | 23.3 | 9.65251 | 59 | 125 | sub-finite |
| JTDD12 | 21.21 | 9.65251 | 86 | 125 | sub-finite |

### Example 5---Tomato

### 1. Hybridization design

A) Experimental materials
   6 parental lines of different origins were adopted in the experiment: Ying-Shi-Da-Hong, Fen-Hong D-80, Bo-Yu 368, Bo-Yu 367, Jin-Xiang-Fan-Zao, Jin-Xiang-Fan-Bao.
B) Experimental methods
   (1) 2 original parental lines of the female group: Ying-Shi-Da-Hong, Fen-Hong D-80
      Hybridization: Ying-Shi-Da-Hong×Fen-Hong D-80, 10 plants in the F₂ segregation generation formed the female group; Bo-Yu 368×Bo-Yu 367, Jin-Xiang-Fan-Zao×Jin-Xiang-Fan-Bao, 1000 individual plants of hybrids formed the male group, and they were hybridized separately with the female group.
   (2) 3 original parental lines of female group: Bo-Yu 367, Jin-Xiang-Fan-Zao, Jin-Xiang-Fan-Bao.
      Hybridization: (Bo-Yu 367xJin-Xiang-Fan-Zao)xJin-Xiang-Fan-Bao, 2000 plants in the segregation generation formed the female group; [(Fen-Hong D-80×Bo-Yu 368) ×(Bo-Yu 367×Jin-Xiang-Fan-Zao)], 100 plants in the segregation generation formed the male group.

### 2. Breeding of colony varieties, cultivars of tomato

A) Materials of the experiment
Intensified breeding of high-quality and comprehensive-character-chosen varieties: Below are the parental lines of crosses. Jia-Fen 17 (from Beijing), Fan-Qie-Dong-Nong 704 (from Heilongjiang), Fan-Qie-Mao-Fen 802 (from Shaanxi), Yu-Fan-Qie 1 (from Henan), Fan-Qie-Zhong-Su 5(from Tiejin), Zhong-Za 11, Zhong-Za 9, Hong-Za 18, Hong-Za 10 (from Chinese Academy of Agricultural Sciences), Jin-Xiang-Fan-Bao, Fan-Qie-Xi-Fen 3, Fen-Hong D-80 (from other areas). Hybridization was conducted between pairs of the above varieties.
B) Experimental methods
The first hybridization:
Jin-Xiang-Fan-Bao×Zhong-Za 11; Fan-Qie-Xi-Fen 3×Fan-Qie-Zhong-Su 5; Jia-Fen 17×Fan-Qie-Dong-Nong 704; Yu-Fan-Qie 1×fen-Hong D-80; Hong-Za 18×Fan-Qie-Mao-Fen 802; Hong-Za 10×Zhong-Za 9.

The secondary hybridization:
(Jin-Xiang-Fan-Bao×Zhong-Za 11) F₁×(Fan-Qie-Xi-Fen 3×Fan-Qie-Zhong-Su 5) F₁, 50 seeds were collected.
(Jia-Fen 17×Fan-Qie-Dong-Nong 704) F₁×(Yu-Fan-Qie 1×Fen-Hong D-80) F₁, 50 seeds were collected.
(Hong-Za 18×Fan-Qie-Mao-Fen 802) F₁×(Hong-Za 10×Zhong-Za 9) F₁, 50 seeds were collected.

The tertiary hybridization:
[(Jin-Xiang-Fan-Bao×Zhong-Za 11) F₁×(Fan-Qie-Xi-Fen 3×Fan-Qie-Zhong-Su 5) F₁] F₁×{[(Jia-Fen 17×Fan-Qie-Dong-Nong 704) F₁×(Yu-Fan-Qie 1×Fen-Hong D-80) F₁] F₁---male 1+ [(Hong-Za 18×Fan-Qie-Mao-Fen 802) F₁×(Hong-Za 10×Zhong-Za 9) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 235 hybrids were obtained from 50 female plants. One self-crossed plant was retained for each hybrid in the subsequent generations up to the 6^{th} generation when a stable population with a wide genetic background was produced. Then each of the plant lines was grown separately for observation of their stability, and the mean weight of their individual fruit, fruit color, growth duration and plant height were investigated as well. The plant lines with the same individual fruit weight, growth duration and plant height were merged into one group to become a colony variety. If the colony was still unstable, a routine roguing could be performed until it became stable and uniform. In total, three colony varieties of JT1, JT2 and JT3 were developed, and their growth duration and plant height are listed below:

| Code | Growth duration (d) | Plant height (cm) |
|---|---|---|
| JT1 | 125 | 97 |
| JT2 | 108 | 78 |
| JT3 | 135 | 105 |

C) Results of the experimental application

Through screening and sorting, 12 colony varieties were developed according to the individual-fruit weight, fruit color and plant height of individual lines of the colony (The parental lines with red or yellow fruit could be mixed intentionally to form multi-color tomatos as well, so that its price would be commercially enhanced). Some of the characteristics are shown in Table 5. Low yield with medium individual-fruit weight is a major limiting factor for the conventional varieties of tomato, and the individual-fruit weight is between 100-200g and the total yield is below 5000Kg/667m² for most varieties. In this experiment, the lines of high individual-plant yield with individual-fruit weights of more than 150g were chosen, and 11 lines were developed. Among them, 7 lines had individual-fruit weights of more than 200g (the highest one had weight of more than 300g and was equivalent to 7000Kg/ 667m²). In comparison to conventional breeding methods, this new strategy greatly increased the probability of obtaining high-yield varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obvious rogue plants. As they belong to a self-pollinating plant, their stability would remain steady during multiplication.

**Table 5: Characterization of colony varieties of soybean**

| Code | Mean individual-fruit weight (g) | Increase over common fruit weight (g) | Plant height (cm) | Growth duration | Fruit color |
|---|---|---|---|---|---|
| JTFQ1 | 155 | -45 | 110 | 136 | pink |
| JTFQ2 | 206 | +6 | 108 | 140 | pink |
| JTFQ3 | 240 | +40 | 86 | 150 | pink |
| JTFQ4 | 202 | +2 | 94 | 120 | pink |
| JTFQ5 | 198 | -2 | 102 | 130 | pink |
| JTFQ6 | 160 | -40 | 84 | 98 | vermeil |
| JTFQ7 | 205 | +5 | 79 | 108 | pink |
| JTFQ8 | 217 | +17 | 74 | 110 | vermeil |
| JTFQ9 | 214 | +14 | 69 | 128 | vermeil |
| JTFQ10 | 164 | - 36 | 64 | 125 | vermeil |
| JTFQ11 | 313 | +113 | 59 | 125 | pink |

### Example 6---Rape

### 1. Hybridization design

A) Experimental materials
   6 parental lines of different origins were utilized in the experiment: H9901, Hua-Za 6, Hua-Za 4, Hua-Za 3, Hua-You (Shuan) 3, Hua-You (Shuan) 4.
B) Experimental methods
   (1) 2 original parental lines of the female group: H9901, Hua-Za 6
      Hybridization: H9901 ×Hua-Za 6, 100 plants in the F₂ segregation generation formed the female group; Hua-Za 4×Hua-Za 3, Hua-You (Shuan) 3×Hua-You (Shuan) 4, 1000 individual plants of hybrids formed the male group, and they were hybridized separately with the female group.
   (2) 3 original parental lines of the female group: Hua-Za 3, Hua-You (Shuan) 3, Hua-You (Shuan) 4.
      Hybridization: (Hua-Za 3×Hua-You (Shuan) 3)×Hua-You (Shuan) 4, 2000 plants in the segregation generation formed the female group; [(H9901 ×Hua-Za 6) ×(Hua-Za 4×Hua-Za 3)], 100 plants in the segregation generation formed the male group.

### 2. Breeding of colony varieties, cultivars of rape

A) Materials of the experiment
Intensified breeding of high-erucil acid-content and comprehensive-character-chosen varieties: Below are the parental lines of crosses. 4 varieties of Mercury, Neptune, Castor and R-588 with a high erucil acid content of more than 53% (introduced from Canada); Indore (introduced from USA); two 'dual-high' varieties in China S87-2127 and S87-2365; three high-linoleic acid and high linolenic acid varieties in China, Zhong-You 821 (by Oil plants Research Institue, Chinese Academy of Agricultural Sciences), Ning-You 7 (by Jiangsu Academy of Agricultural Sciences), Wang-You 17 (from Hubei); Low-thioglycoside variety Wan-You 6 (by Anhui Academy of Agricultural Sciences); introduced 'dual-high' variety Gotarshiji. Hybridization was conducted between pairs of the above varieties.
B) Experimental methods
The first hybridization:
Mercury×S87-2127; Neptune×S87-2365; Castor×Zhong-You 821; Indore×Ning-You 7; Gotarshiji×Wang-You 17; R-588×Wan-You 6.

The secondary hybridization:
(Mercury×S87-2127) ×(Neptune×S87-2365), 50 seeds were collected.
(CastorxZhong-You 821)×(Indore×Ning-You 7), 50 seeds were collected.
(Gotarshiji×Wang-You 17) ×(R-588×Wan-You 6), 50 seeds were collected

The tertiary hybridization:
[(Mercury×S87-2127)×(Neptune×S87-2365)]×{[(Castor×Zhong-You821)× (Indore×Ning-You 7)]---male 1+ [(Gotarshiji×Wang-You 17) ×(R-588×Wan-You 6)]---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 218 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Growth duration (d) | Plant height (cm) |
|---|---|---|
| JT1 | 234 | 158 |
| JT2 | 239 | 164 |
| JT3 | 243 | 169 |

C) Results of the experimental application
After being stable through self-crossing, all individual lines were planted in the experimental field of Changjiang University in the Hubei province of China, and their dates of bolting and maturity, plant height at bolting and maturing stages and individual-plant yield were investigated. Through screening and sorting by computer, 12 colony varieties were developed according to the individual-plant yields of individual lines of the colony (those lines with individual-plant yield of less than 30g were eliminated). Some of their characteristics are shown in Table 6. As they were taken for a major object of selection, their yields were enhanced greatly, with 8 lines (accounting for 67%) exceeding the control group in yield. Such big an increase in yield was seldom seen from conventional breeding of rape. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As rape belongs to a frequent-cross-pollinating plant and has a self-seed-setting rate of more than 80%, their stability remained unchanged over the short term during the multiplication. After purification and rejuvenation through isolated planting of breeder's stock seeds, the colony varieties could be multiplied for their seeds.

**Table 6 Characterization of colony varieties of rape**

| Code | Seeding date (m-d) | Bolting date (m-d) | Maturing date (m-d) | Plant height at maturity (cm) | Mean individual-plant yield (g) |
|---|---|---|---|---|---|
| JTYC1 | 09-18 | 01-23 | 05-05 | 167 | 38.45 |
| JTYC2 | 09-18 | 01-26 | 05-07 | 165 | 35.84 |
| JTYC3 | 09-18 | 01-27 | 05-10 | 169 | 34.18 |
| JTYC4 | 09-18 | 01-29 | 05-11 | 172 | 44.08 |
| JTYC5 | 09-18 | 02-01 | 05-13 | 172 | 39.10 |
| JTYC6 | 09-18 | 02-03 | 05-14 | 173 | 44.38 |
| JTYC7 | 09-18 | 02-10 | 05-18 | 175 | 44.11 |
| JTYC8 | 09-18 | 02-15 | 05-18 | 169 | 40.31 |
| JTYC9 | 09-18 | 02-16 | 05-18 | 167 | 31.44 |
| JTYC10 | 09-18 | 02-17 | 05-19 | 156 | 30.11 |
| JTYC11 | 09-18 | 02-18 | 05-19 | 154 | 32.01 |
| JTYC12 | 09-18 | 02-19 | 05-21 | 162 | 34.02 |
| Zhong-You 821 | 09-18 | 01-22 | 05-15 | 165 | 34.15 |

### Example 7---Cotton

### 1. Hybridization design

A) Experimental materials
   6 parental lines of different origins were utilized in the experiment: Zhong-Mian-Suo 24, Zhong-Mian-Suo 35, Zhong-Mian-Suo 36, Zhong-Mian-Suo 19, Yu-Mian 19, Han-Dan 284.
B) Experimental methods
   (1) 2 original parental lines of the female group: Zhong-Mian-Suo 24, Zhong-Mian-Suo 35
      Hybridization: Zhong-Mian-Suo 24×Zhong-Mian-Suo 35, 10 plants in the F₂ segregation generation formed the female group; Zhong-Mian-Suo 36×Zhong-Mian-Suo 19, Yu-Mian 19xHan-Dan 284, 100 individual plants of hybrids formed the male group, and they were hybridized separately with the female group.
   (2) 3 original parental lines of the female group: Zhong-Mian-Suo 19, Yu-Mian 19, Han-Dan 284.
      Hybridization: (Zhong-Mian-Suo 19×Yu-Mian 19)×Han-Dan 284, 200 plants in the segregation generation formed the female group; [(Zhong-Mian-Suo 35×Zhong-Mian-Suo 36) ×(Zhong-Mian-Suo 19×Yu-Mian 19)], 100 plants in the segregation generation formed the male group.

### 2. Breeding of colony varieties, cultivars of cotton

A) Materials of the experiment
Intensified breeding of comprehensive-character-chosen varieties: Below are the parental lines of crosses. Zhong-Mian-Suo 38, Zhong-Mian-Suo 23, Zhong-Mian-Suo 27, Zhong-Mian-Suo 29 from Cotton Research Institute, Chinese Academy of Agricultural Sciences; E-Kang-Mian 7 and E-Kang-Mian 3 from Hubei; Yu-Mian 12 and Yu-Zao 275 from Henan; Ji-Mian 21 and Ji 668 from Hebei; Su-Mian 12 from Jiangsu and Chuan-Mian 239 from Sichuan.
Hybridization was conducted between pairs of the above varieties.
B) Experimental methods
The first hybridization:
Zhong-Mian-Suo 38×Su-Mian 12; Ji-Mian 21×Ji 668; Zhong-Mian-Suo 23xE-Kang-Mian 3; Yu-Zao 275xE-Kang-Mian 7; Zhong-Mian-Suo 27xYu-Mian 12; Zhong-Mian-Suo 29×Chuan-Mian 239

The secondary hybridization:
(Zhong-Mian-Suo 38×Su-Mian 12) F₁ ×(Ji-Mian 21×Ji 668) F₁, 50 seeds were collected.
(Zhong-Mian-Suo 23×E-Kang-Mian 3) F₁×(Yu-Zao 275×E-Kang-Mian 7) F₁, 50 seeds were collected.
(Zhong-Mian-Suo 27×Yu-Mian 12) F₁×(Zhong-Mian-Suo 29×Chuan-Mian 239) F₁, 50 seeds were collected

The tertiary hybridization:
[(Zhong-Mian-Suo 38×Su-Mian 12) F₁ ×(Ji-Mian 21×Ji 668) F₁] F₁×{[(Zhong-Mian-Suo 23×E-Kang-Mian 3) F₁×(Yu-Zao 275×E-Kang-Mian 7) F₁] F₁---male 1 + [(Zhong-Mian-Suo 27×Yu-Mian 12) F₁×(Zhong-Mian-Suo 29×Chuan-Mian 239) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 220 hybrids were obtained from 50 female plants. One self-crossed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Growth duration (d) | Plant height (cm) |
|---|---|---|
| JT1 | 110 | 78 |
| JT2 | 125 | 89 |
| JT3 | 135 | 100 |

C) Results of the experimental application

Through screening and sorting, 12 colony varieties were developed according to the growth duration, plant height and yield in turn of individual lines of the colony. Some of their characteristics are shown in Table 7. Low yield is a major limiting factor for the conventional varieties of cotton, and the ginned cotton yield is below 90Kg/667m² for most varieties and few varieties have a yield of more than this figure. In the breeding period, even fewer breeding lines have a high ginned cotton yield. In this experiment, 7 lines (accounting for 58%) had a ginned cotton yield of more than 90Kg/667m², and one line arrived at 100 Kg/667m². In comparison to conventional breeding methods, this new strategy greatly increased the probability of obtaining high-yield varieties. The uniformity of the colony varieties could basically be fulfilled through selection on computer. Their peculiarity and uniformity depended on the accuracy of measurement and the allowed extent of variation, and so they could be artificially controlled. They just needed proper selection and removal of those obviously rogue plants. As cotton belongs to a frequent-cross-pollinating plant and has a self-seed-setting rate of more than 80%, their stability would remain unchanged over a short term during multiplication. After purification and rejuvenation through isolated planting of breeder's stock seeds, the colony varieties could be multiplied for their seeds.

**Table 7 Characterization of colony varieties of cotton**

| Code | yield (kg/667m²) | Increase over common variety (kg/667m²) | Plant height (cm) | Growth duration (d) |
|---|---|---|---|---|
| JTMH1 | 76 | -14 | 80 | 125 |
| JTMH2 | 94 | +4 | 88 | 130 |
| JTMH3 | 99 | +9 | 89 | 135 |
| JTMH4 | 82 | -8 | 74 | 130 |
| JTMH5 | 90 | 0 | 102 | 120 |
| JTMH6 | 93 | +3 | 94 | 110 |
| JTMH7 | 108 | +18 | 89 | 122 |
| JTMH8 | 98 | +8 | 74 | 128 |
| JTMH9 | 86 | -4 | 69 | 124 |
| JTMH10 | 96 | +6 | 64 | 112 |
| JTMH 11 | 84 | - 6 | 59 | 109 |
| JTMH12 | 84 | -6 | 86 | 70 |

### Example 8---Chilli Pepper

### 1. Hybridization design

A) Experimental materials
   6 parental lines of different origins were utilized in the experiment: Zhong-Jiao 6, Zhong-Jiao 5, Zhong-Jiao 11, Ning-Jiao 5, B Te-Zao, Ha-Jiao 3.
B) Experimental methods
   (1) 2 original parental lines of the female group: Zhong-Jiao 6, Zhong-Jiao 5
      Hybridization: Zhong-Jiao 6×Zhong-Jiao 5, 1000 plants in the F₂ segregation generation formed the female group; Zhong-Jiao 11×Ning-Jiao 5, B Te-Zao×Ha-Jiao 3, 1000 individual plants of the hybrids formed the male group, and they were hybridized separately with the female group.
   (2) 3 original parental lines of the female group: Ning-Jiao 5, B Te-Zao, Ha-Jiao 3
      Hybridization: (Ning-Jiao 5×B Te-Zao)×Ha-Jiao 3, 2000 plants in the segregation generation were the female group; [(Zhong-Jiao5×Zhong-Jiao 11) ×(Ning-Jiao 5×B Te-Zao)], 2000 plants in the segregation generation were the male group.

### 2. Breeding of colony varieties, cultivars of chilli pepper

A) Materials of experiment
Intensified breeding of high-quality and comprehensive-character-chosen varieties: Below are the parental lines of crosses. Shen-Jiao 4 and Shen-Jiao 6 from Liaoning; Jiang-Shu 1, Jiang-Shu 5 and Jiang-Shu 6 from Jiangsu; La-You 1 and YUe-Jiao 1 from Guangdong; Xiang-Jiao 6 and Xiang-Jiao 9 from Hunan; Yu-Jiao 5 from Chongqing; Zhong-Jiao 10 and Zhong-Jiao 7 from the Chinese Academy of Agricultural Sciences. Hybridization was conducted between pairs of the above varieties.
B) Experimental methods
The first hybridization:
Shen-Jiao 4×Zhong-Jiao 10; Jiang-Shu 6×Yu-Jiao 5; Shen-Jiao 6×Yue-Jiao 1; Xiang-Jiao 9×La-You 1; Jiang-Shu 1×Xiang-Jiao 6; Jiang-Shu 5×Zhong-Jiao 7

The secondary hybridization:
(Shen-Jiao 4×Zhong-Jiao 10) F₁ ×(Jiang-Shu 6×Yu-Jiao 5) F₁, 50 seeds were collected.
(Shen-Jiao 6×Yue-Jiao 1) F₁×(Xiang-Jiao 9×La-You 1) F₁, 50 seeds were collected.
(Jiang-Shu 1×Xiang-Jiao 6) F₁×(Jiang-Shu 5×Zhong-Jiao 7) F₁, 50 seeds were collected

The tertiary hybridization:
[(Shen-Jiao 4×Zhong-Jiao 10) F₁ ×(Jiang-Shu 6×Yu-Jiao 5) F₁] F₁×{[(Shen-Jiao 6×Yue-Jiao 1) F₁×(Xiang-Jiao 9×La-You 1) F₁] F₁---male 1 + [(Jiang-Shu 1×Xiang-Jiao 6) F₁×(Jiang-Shu 5×Zhong-Jiao 7) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 220 hybrids were obtained from 50 female plants. One self-crossed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration, plant height and fruit shape were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Growth duration (d) | Plant height (cm) |
|---|---|---|
| JT1 | 128 | 57 |
| JT2 | 118 | 52 |
| JT3 | 109 | 48 |

C) Results of the experimental application

Through screening and sorting, 10 colony varieties were developed according to the plant height, fruit shape and maturity in turn of individual lines of the colony. Some of their characteristics are shown in Table 8. Unstable yield caused by pests and diseases is a major limiting factor for the conventional varieties of chilli pepper, and the yield is generally around 2500Kg/667m² for most varieties. In this experiment, the harm of pests and diseases was reduced due to multiple genotypes and the selection in yield was intensified. As a result, the yield of 10 lines was enhanced, and 7 lines (accounting for 70%) had yields of more than 2500Kg/667m². No significant yield reduction was observed for the other lines. In comparison to conventional breeding methods, this new strategy greatly increased the probability of obtaining high-yield varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As chilli pepper belongs to a frequent-cross-pollinating plant and has a self-seed-setting rate of more than 80%, their stability would remain unchanged over the short term during multiplication. After purification and rejuvenation through isolated planting of breeder's stock seeds, the colony varieties could be multiplied for seeds. In comparison to hybrid seed production, this new strategy is obviously simple and practical. It only needs self-crossing of lines within a colony for their multiplication and the lines will be mixed as required.

**Table 8 Characterization of colony varieties of chilli pepper**

| Code | Fruit shape | Yield (kg/667m²) | Increase over common varieties (kg/667m²) | Plant height (cm) | Growth duration (d) |
|---|---|---|---|---|---|
| JTLJ1 | horn | 2804 | +304 | 50 | 127 |
| JTLJ2 | horn | 3684 | +1184 | 56 | 127 |
| JTLJ3 | lantern | 2972 | +472 | 48 | 127 |
| JTLJ4 | horn | 2720 | +220 | 40 | 125 |
| JTLJ5 | horn | 2860 | +360 | 44 | 118 |
| JTLJ6 | lantern | 2670 | +170 | 46 | 120 |
| JTLJ7 | lantern | 2348 | -152 | 39 | 116 |
| JTLJ8 | horn | 2498 | -2 | 42 | 118 |
| JTLJ9 | lantern | 2446 | -54 | 45 | 117 |
| JTLJ10 | horn | 2824 | +324 | 58 | 120 |

### Example 9--- Flowering Chinese Cabbage (Brassica campestris L. ssp. Chinensis var. utilis Tsen, et, Lee)

### 1. Hybridization design

A) Experimental materials
   6 parental lines of different origins were utilized in the experiment: Xiang-Gang 31, Qing-Bao 40, You-Lu 70 Tian, You-Qing 49, You-Qing 12, Te-Qing-Chi-Xin 4.
B) Experimental methods
   (1) 2 original parental lines of the female group: Xiang-Gang 31, Qing-Bao 40. Hybridization:
      Xiang-Gang 3 1×Qing-Bao 40, 10 plants in the F₂ segregation generation formed the female group; You-Lu 70 Tian×You-Qing 49, You-Qing 12×Te-Qing-Chi-Xin 4, 100 individual plants of the hybrids formed the male group, and they were hybridized separately with the female group.
   (2) 3 original parental lines of the female group:
      You-Qing 49, You-Qing 12, Te-Qing-Chi-Xin 4
      Hybridization: (You-Qing 49×You-Qing 12) × Te-Qing-Chi-Xin 4, 2000 plants in the segregation generation formed the female group; [(Qing-Bao 40×You-Lu 70 Tian) × (You-Qing 49×You-Qing 12)], 100 plants in the segregation generation formed the male group.

### 2. Breeding of colony varieties, cultivars of flowering chinese cabbage

A) Materials of experiment
Breeding of high-quality and comprehensive-character-chosen varieties: Listed below are the parental lines of crosses and they originate from the Guangdong province. Te-Qing-Chi-Xin 4, Chi-Xin 29 and Sui-Qing 1 of dark-green type; You-Qing 12, Si-Jiu 19, You-Qing 49, Lu-Bao 70, Qing-Bao 40 of light-green type; You-Lu 70, Chi-Xin 2, You-Qing 50 of glossy-green type; Si-Jiu-Cai-Xin of yellow-green type. Hybridization was conducted between pairs of the above varieties.
B) Experimental methods
The first hybridization:
Te-Qing-Chi-Xin 4×You-Qing 50; You-Qing 49×Chi-Xin 2; Qing-Bao 40×Sui-Qing 1; Lu-Bao 70×You-Lu 70; You-Qing 12×Si-Jiu 19; Si-Jiu-Cai-Xin×Chi-Xin 29

The secondary hybridization:
(Te-Qing-Chi-Xin 4×You-Qing 50) F₁ ×(You-Qing 49×Chi-Xin 2) F₁, 50 seeds were collected.
(Qing-Bao 40×Sui-Qing 1) F₁×(Lu-Bao 70×You-Lu 70) F₁, 50 seeds were collected.
(You-Qing 12×Si-Jiu 19) F₁×(Si-Jiu-Cai-Xin×Chi-Xin 29) F₁, 50 seeds were collected

The tertiary hybridization:
[(Te-Qing-Chi-Xin 4×You-Qing 50) F₁ ×(You-Qing 49×Chi-Xin 2) F₁] F₁×{[(Qing-Bao 40×Sui-Qing 1) F₁×(Lu-Bao 70×You-Lu 70) F₁] F₁---male 1 + [(You-Qing 12×Si-Jiu 19) F₁×(Si-Jiu-Cai-Xin×Chi-Xin 29) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 2000 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Days to initial harvest (d) | Plant height (cm) |
|---|---|---|
| JT1 | 28 | 25 |
| JT2 | 38 | 32 |
| JT3 | 50 | 40 |

C) Results of the experimental application
Through screening and sorting, 10 colony varieties were developed according to the initial harvest, plant height and leaf color in turn of individual lines of the colony. Some of their characteristics are shown in Table 9. Unstable yield caused by pests and diseases is a major limiting factor for the conventional varieties of flowering Chinese cabbage, and the yield is generally around 700-1200Kg/667m² for most varieties. In this experiment, the harm of pests and diseases was reduced in the multi-genotype lines and the selection in yield was intensified. As a result, the yield of 10 lines was enhanced, and 6 lines (accounting for 60%) had yields of more than 1200Kg/667m². No significant yield reduction was observed for the other lines. In comparison to conventional breeding methods, this new strategy greatly increased the probability of obtaining high-yield varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As flowering Chinese cabbage belongs to a frequent-cross-pollinating plant and has a self-seed-setting rate of more than 80%, their stability would remain unchanged over the short term during their multiplication. After purification and rejuvenation through isolated planting of breeder's stock seeds, the colony varieties could be multiplied for their seeds. In comparison to hybrid seed production, this new strategy is obviously simple and practical. It only needs self-crossing of lines within a colony for their multiplication and the lines will be mixed as required.

**Table 9 Characterization of colony varieties of flowering Chinese cabbage**

| Code | Yield (kg/667m²) | Increase over common varieties (kg/667m²) | Plant height (cm) | Days to initial harvest (d) |
|---|---|---|---|---|
| JTCX1 | 1365 | +165 | 30 | 30-35 |
| JTCX2 | 1434 | +234 | 28 | 35-40 |
| JTCX3 | 1332 | +132 | 25 | 30-36 |
| JTCX4 | 1323 | +123 | 30 | 30-35 |
| JTCX5 | 1020 | -80 | 24 | 28-32 |
| JTCX6 | 1102 | -98 | 23 | 28-33 |
| JTCX7 | 1308 | +108 | 35 | 30-35 |
| JTCX8 | 1508 | +308 | 35 | 40-45 |
| JTCX9 | 1196 | -4 | 23 | 30-35 |
| JTCX10 | 1169 | -31 | 25 | 28-30 |

### Example 10--- Eggplant

### 1. Hybridization design

A) Experimental materials
   6 parental lines of different origins were utilized in the experiment: Hei-Bao-Fu-Qiu-Qie, Hei-Hu-Zao-Qie, Lu-Qie 1, Lu-Qie 3, Lu-Qie 4, Bai-Qie 1.
B) Experimental methods
   (1) 2 original parental lines of the female group: Hei-Bao-Fu-Qiu-Qie, Hei-Hu-Zao-Qie.
      Hybridization: Hei-Bao-Fu-Qiu-Qiex Hei-Hu-Zao-Qie, 10 plants in the F₂ segregation generation were the female group; Lu-Qie 1 × Lu-Qie 3, Lu-Qie 4×Bai-Qie 1, 120 individual plants of the hybrids were the male group and they were hybridized separately with female group.
   (2) 3 original parental lines of the female group: Lu-Qie 3, Lu-Qie 4, Bai-Qie 1
      Hybridization: (Lu-Qie 3xLu-Qie 4) × Bai-Qie 1, 2000 plants in the segregation generation formed the female group; [(Hei-Hu-Zao-QiexLu-Qie 1) ×(Lu-Qie 3×Lu-Qie 4)], 100 plants in the segregation generation formed the male group.

### 2. Breeding of colony varieties, cultivars of eggplant

A) Materials of the experiment
Breeding of high-quality and comprehensive-character-chosen varieties: Below are the parental lines of crosses and they had different fruit shape and color and were from the Guangdong province. Zi-Hei 2, Hei-Bao-Fu-Qiu-Qie, Hei-Hu-Zao-Qie of the dark-purple type; Tai-Ke-Zi-Yuan-Qie and American Hei-Jin of the dark-purple and round type; 9318 Chang-Qie, Jia-Li-Chang-Qie, Ji-Nan 94-1, Chang-Hong 2 and Long-Feng-Qie-Zi of the purple and long type; Bang-Lu-Qie and Lu-Qie 3 of the glossy-green type. Hybridization was conducted between pairs of the above varieties.
B) Experimental methods
The first hybridization:
Zi-Hei 2×Lu-Qie 3; American Hei-Jin×Bang-Lu-Qie; Ji-Nan 94-1×Hei-Hu-Zao-Qie; Jia-Li-Chang-Qie×Chang-Hong 2; 9318 Chang-Qie×Tai-Ke-Zi-Yuan-Qie; Long-Feng-Qie-Zi×Hei-Bao-Fu-Qiu-Qie;

The secondary hybridization:
(Zi-Hei 2×Lu-Qie 3) F₁ ×(American Hei-Jin×Bang-Lu-Qie) F₁, 50 seeds were collected.
(Ji-Nan 94-1×Hei-Hu-Zao-Qie) F₁×(Jia-Li-Chang-Qie×Chang-Hong 2) F₁, 50 seeds were collected.
(9318 Chang-Qie×Tai-Ke-Zi-Yuan-Qie) F₁×(Long-Feng-Qie-Zi×Hei-Bao-Fu-Qiu-Qie) F₁, 50 seeds were collected

The tertiary hybridization:
[(Zi-Hei 2×Lu-Qie 3) F₁ x(American Hei-JinxBang-Lu-Qie) F₁] F₁×{[(Ji-Nan 94-1×Hei-Hu-Zao-Qie) F₁×(Jia-Li-Chang-Qie×Chang-Hong 2) F₁] F₁---male 1 + [(9318 Chang-QiexTai-Ke-Zi-Yuan-Qie) F₁×(Long-Feng-Qie-Zi×Hei-Bao-Fu-Qiu-Qie) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. A total of 2000 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration, plant height and fruit shape and color were investigated as well. The plant lines with the same growth duration, plant height and fruit type were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Days to initial plucking (d) | Plant height (cm) |
|---|---|---|
| JT1 | 108 | 78 |
| JT2 | 112 | 85 |
| JT3 | 110 | 92 |

C) Results of the experimental application

Through screening and sorting, 10 colony varieties were developed according to the initial plucking, plant height and fruit type in turn of individual lines of the colony. Some of their characteristics are shown in Table 10. Unstable yield caused by pests and diseases is a major limiting factor for the conventional varieties of eggplant, and the yield is generally around 4000-4500Kg/667m² for most varieties. In this experiment, the harm of pests and diseases was reduced by multi-genotype lines and the selection in yield was intensified. As a result, the yield of 10 lines was enhanced, and 6 lines (accounting for 60%) had yields of more than 4500Kg/667m². In comparison to conventional breeding methods, this new strategy greatly increased the probability of obtaining high-yield varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As eggplant belongs to a frequent-cross-pollinating plant and has a self-seed-setting rate of more than 80%, their stability would remain unchanged over the short term during multiplication. After purification and rejuvenation through isolated planting of breeder's stock seeds, the colony varieties could be multiplied for their seeds. In comparison to hybrid seed production, this new strategy is obviously simple and practical. It only needs self-crossing of lines within a colony for their multiplication and the lines will be mixed as required.

**Table 10 Characterization of colony varieties of eggplant**

| Code | Yield (kg/667m²) | Increase over common varieties (kg/667m²) | Plant height (cm) | Day to initial plucking (d) | Fruit shape and color |
|---|---|---|---|---|---|
| JTQZ1 | 4365 | -135 | 70 | 100 | long, dark-purple |
| JTQZ2 | 4834 | +334 | 90 | 110 | long, green |
| JTQZ3 | 4923 | +423 | 95 | 102 | oval,purple |
| JTQZ4 | 4236 | -264 | 75 | 110 | oval, green |
| JTQZ5 | 4402 | -98 | 65 | 108 | long, green |
| JTQZ6 | 4720 | +220 | 90 | 112 | round, dark-purple |
| JTQZ7 | 4980 | +480 | 85 | 104 | oval, purple |
| JTQZ8 | 4480 | -20 | 75 | 113 | long, green |
| JTQZ9 | 5069 | +569 | 88 | 115 | oval, green |
| JTQZ10 | 5096 | +596 | 86 | 114 | long, dark-purple |

### Example 11--- Cabbage Mustard

### 1. Hybridization design

A) Experimental materials
   6 parental lines of different origins were utilized in the experiment: Zhong-Hua-Jie-Lan, Pu-Tong-Jie-Lan-1, Dong-Fang-Jian-Jie-Lan, Bai-Hua-Jie-Lan, Xi-Ye-Za-Jie-Lan, Zhou-Ye-Zao-Jie-Lan.
B) Experimental methods
   (1) 2 original parental lines of the female group: Zhong-Hua-Jie-Lan, Pu-Tong-Jie-Lan-1.
      Hybridization: Zhong-Hua-Jie-Lan×Pu-Tong-Jie-Lan-1 10 plants in the F₂ segregation generation were the female group; Dong-Fang-Jian-Jie-LanxBai-Hua-Jie-Lan, Xi-Ye-Za-Jie-LanxZhou-Ye-Zao-Jie-Lan, 120 individual plants of the hybrids were the male group and they were hybridized separately with the female group.
   (2) 3 original parental lines of the female group:
      Bai-Hua-Jie-Lan, Xi-Ye-Za-Jie-Lan, Zhou-Ye-Zao-Jie-Lan.
      Hybridization: (Bai-Hua-Jie-Lan×Xi-Ye-Za-Jie-Lan) × Zhou-Ye-Zao-Jie-Lan, 1500 plants in segregation generation formed the female group; [(Pu-Tong-Jie-Lan-1 ×Dong-Fang-Jian-Jie-Lan)×(Bai-Hua-Jie-Lan×Xi-Ye-Za-Jie-Lan)], 1500 plants in the segregation generation formed the male group.

### 2. Breeding of colony varieties, cultivars of cabbage mustard

A) Materials of experiment
Breeding of high-quality and comprehensive-character-chosen varieties: different series of varieties were adopted as parental lines of crosses. Xiang-Gang-Bai-Hua-Jie-Lan, Xi-Ye-Zao-Jie-Lan, Zhou-Ye-Zao-Jie-Lan of early-maturing type; Tai-Wang-Zhong-Hua, Zhong-Chi-Jie-Lan, Xiang-Gang-Zhong-Hua, He-Tan-Jie-Lan and Zhong-Hua-Jie-Lan of medium-maturing type; Zhou-Ye-Chi-Jie-Lan, Dong-Fang-Jian-Jie-Lan, Pu-Tong-Jie-Lan-1, Chi-Hua-Jie-Lan of late-maturing type. Hybridization was conducted between pairs of the above varieties.
B) Experimental methods
The first hybridization:
Xiang-Gang-Bai-Hua-Jie-Lan×Pu-Tong-Jie-Lan-1;
Xiang-Gang-Zhong-Hua×Dong-Fang-Jian-Jie-Lan; Chi-Hua-Jie-Lan×Zhou-Ye-Zao-Jie-Lan;
He-Tan-Jie-Lan×Zhou-Ye-Chi-Jie-Lan; Tai-Wang-Zhong-Hua×Zhong-Chi-Jie-Lan;
Zhong-Hua-Jie-Lan×Xi-Ye-Zao-Jie-Lan;

The secondary hybridization:
(Xiang-Gang-Bai-Hua-Jie-Lan×Pu-Tong-Jie-Lan-1) F₁ ×(Xiang-Gang-Zhong-Hua×Dong-Fang-Jian-Jie-Lan) F₁, 50 seeds were collected.
(Chi-Hua-Jie-Lan×Zhou-Ye-Zao-Jie-Lan) F₁×(He-Tan-Jie-Lan×Zhou-Ye-Chi-Jie-Lan) F₁, 50 seeds were collected.
(Tai-Wang-Zhong-Hua×Zhong-Chi-Jie-Lan) F₁×(Zhong-Hua-Jie-Lan×Xi-Ye-Zao-Jie-Lan) F₁, 50 seeds were collected

The tertiary hybridization:
[(Xiang-Gang-Bai-Hua-Jie-Lan×Pu-Tong-Jie-Lan-1)F₁ ×(Xiang-Gang-Zhong-Hua×Dong-Fang-J ian-Jie-Lan)F₁] F₁× {[(Chi-Hua-Jie-Lan×Zhou-Ye-Zao-Jie-Lan) F₁
×(He-Tan-Jie-Lan×Zhou-Ye-Chi-Jie-Lan)F₁]F₁ ---male1+[(Tai-Wang-Zhong-Hua×Zhong-Chi-Ji e-Lan)F₁ ×(Zhong-Hua-Jie-Lan×Xi-Ye-Zao-Jie-Lan) F₁] F₁---male 2}
Note: in the above notations, the female is placed in front and the male in back.
For the tertiary hybridization, the individual plants of each female parent were pollinated with the mixed pollens of male 1 and male 2, and 4 to 5 hybrid seeds were acquired from each plant. After three hybridization, a total of 2000 hybrids were obtained from 50 female plants. One selfed plant was retained for each hybrid in the subsequent generations up to the 6^{th} generation when a stable population with a wide genetic background was produced. Then each of the plant lines was grown separately for observation of their stability, and their growth duration and plant height were investigated as well. The plant lines with the same growth duration and plant height were merged into a group to become a colony variety. If the colony was still unstable, a routine roguing could be performed until it became stable and uniform. In total, three colony varieties of JT1, JT2 and JT3 were developed, and their growth duration and plant height are listed below:

| Code | Days to initial harvest(d) | Plant Height (cm) |
|---|---|---|
| JT1 | 30 | 28 |
| JT2 | 37 | 31 |
| JT3 | 40 | 38 |

C) Result of the experimental application

Through screening and sorting, 10 colony varieties were developed according to the initial harvest, plant height and leaf color in turn of individual lines of the colony. Some of their characteristics are shown in Table 11. Unstable yield caused by pests and diseases is a major limiting factor for the conventional varieties of cabbage mustard, and the yield is generally around 1000-1500Kg/667m² for most varieties. In this experiment, the harm of pests and diseases was reduced by multi-genotype lines and the selection in yield was intensified. As a result, the yield of 10 lines was enhanced, and 5 lines (accounting for 50%) had yields of more than 1500Kg/667m². In comparison to conventional breeding methods, this new strategy greatly increased the probability of obtaining high-yield varieties. The uniformity of colony varieties could be basically fulfilled through selection on computer. Their peculiarity and uniformity depended on the accuracy of measurement and the allowed extent of variation, and so they could be artificially controlled. They just needed proper selection and removal of those obviously rogue plants. As cabbage mustard belongs to a frequent-cross-pollinating plant and has a self-seed-setting rate of more than 80%, their stability would remain unchanged over the short term during multiplication. After purification and rejuvenation through isolated planting of breeder's stock seeds, the colony varieties could be multiplied for their seeds. In comparison to hybrid seed production, this new strategy is obviously simple and practical. It only needs self-crossing of lines within a colony for their multiplication and the lines will be mixed as required.

**Table 11; Characterization of colony varieties of cabbage mustard**

| Code | Yield (kg/667m²) | Increase over common varieties (kg/667m²) | Plant height (cm) | Days to initial harvest (d) |
|---|---|---|---|---|
| JTJL1 | 1556 | +56 | 35 | 32-37 |
| JTJL2 | 1643 | +157 | 28 | 33-38 |
| JTJL3 | 1544 | -f-44 | 29 | 30-38 |
| JTJL4 | 1528 | +28 | 32 | 31-36 |
| JTJL5 | 1250 | -250 | 24 | 30-5 |
| JTJL6 | 1323 | -177 | 33 | 28-33 |
| JTJL7 | 1498 | -2 | 32 | 30-35 |
| JTJL8 | 1710 | +210 | 30 | 40-45 |
| JTJL9 | 1498 | -2 | 29 | 30-35 |
| JTJL10 | 1475 | -25 | 28 | 29-31 |

### Industrial Application of Genetic Diversity Breeding

In light of bio-diversity and durable development: Due to the traditional manner of planting single-genotype varieties and the conventional breeding strategy of selecting the best from the best, the genetic backgrounds of crop plants have been becoming increasingly narrow in the past decades, and they have also become increasingly weak in their resistance to pests, diseases and other stresses. As a result, the life-span of varieties has become increasingly short for crop plants, from at least 5 years or even up to more than a decade in the past for a given variety, to around 4 years on average for modem varieties. The extinction of living species has sped up and bio-diversity has declined rapidly ( Dabao Zhu, Diversity and bio-diversity in forest woods breeding, 1994, 2 (3): 157-161). In view of coordination between living species and environment and diversified demands of human being, a new strategy of exploiting multi-genotypes is thus urgently needed to develop for improved production of crop plants. The core of this new strategy is that an elite group is more important than perfect individuals.

Views from the perspective of leaping development of crop breeding: making advancements in crop improvements is becoming increasingly harder, even though a great number of varieties and combinations have been developed. In the past, improvements in yield, quality and disease-resistance arrived at a very high level. For instance, the current average yield of rice in China has been increased to more than 6000 Kg/ha from a level of around 3000 Kg/ha in the past. It is increasingly difficult to achive breakthroughs in increasing yield and in other respects for current two types of crops. Thus a new strategy of breeding different type of crops needs to be developed.

Views from the perspective of seed production of crop plants: Farmers can reserve seeds of conventional varieties of crops for planting, but they can only use the seeds of hybrid combinations for just one generation. With the development of industrialization in agriculture, seed enterprises, driven by economical returns, usually produce hybrid seeds, although the hybrid combinations of crops are generally inferior to conventional varieties in product quality. The new strategy in this invention is simple and practical. It has less reliance on the experience of the breeders. It exploits the genetic diversity of living species and will promote the durable development of human beings. It is also suitable for commercialization of this technology. Equally important, it opens a new route for crop breeding and it will have tremendous significance for leaping advancement in breeding science. As colony varieties are characterized by high quality, adaptability and resistance to disease, management of their cultivation is quite easy for farmers, and a high demand can be expected for them in the marketplace. With their release and extention into the market place, this new breeding strategy and these new types of crops will be widely recognized, and will greatly promote the development of breeding science and agriculture.

Views from the perspective of the adaptability of living species to the environment: In comparison to multi-genotype varieties, single-genotype varieties perform poorly in adaptability to the environment and in resistance to pests and diseases. A good demonstration can be presented for the success in the control of diseases by alternate planting of blast-resistant multi-lines of rice in Yunna province of China (Youyong Zhu, et al., Nature, 406: 718-722).

Views from the perspective of the consumption needs of human beings for crops: As a matter of fact, a lot of crops are consumed in mixed forms which contain many genotypes. The colony varieties in this invention will directly provide multi-genotype products.

Views from the perspective of the demands of farmers: The seeds of colony varieties can be reserved by farmers for planting for one or two generations, so they will be warmly welcomed by farmers. However, seed companies can still make profits through controlling their stock seeds, and so this strategy will also be accepted by the companies.

Through years of effort, a new breeding strategy for developing comprehensive-character-chosen colony varieties or cultivars has been invented. This is also a breeding strategy for exploiting genetic diversity. The new strategy in the present invention will be a creative breakthrough in crop breeding. The colony varieties have good adaptability and high resistance to pests and diseases and will be broadly exploited for their application in the future.

## Claims

1. A method for breeding colony varieties of crops, **characterized by**:
hybridizing a female group with a male group to produce a primary population of colony variety, wherein said female group consists of individual plants in a segregation population or self-crossed descendants of early segregation generations which is obtained by hybridizing pairs of parental plants with different desired characteristics to produce population F1, and then hybridizing pairs of F1 to produce the segregation population, and wherein said male group consists of homozygous breeding lines, varieties, F₁, heterozygous plants in the segregation generations or individual plants produced in the same manner for the production as the female group.

2. The method for breeding colony varieties of crops according to Claim 1, **characterized in that** said method comprises the following steps:
1) Hybridization: a female group consisting of individual plants in a segregation population or their self-crossed descendants in the early segregation generations is produced by hybridizing pairs of parental plants with different predetermined characteristics to produce F1, and hybridizing pairs of F1 to produce the segregation population; a male group is made up of homozygous breeding lines, varieties, F₁, heterozygous plants in the segregation generations or individual plants produced in the same manner as the production of the female group; a primary population of colony varieties is produced after a female group is hybridized with a male group;
2) Self-crossing for stabilization: determining the number of individual plants of descendants of each hybrid for the primary population of a colony variety, according to a predetermined breeding goal and multiplication coefficients of the crop species; and then planting individual lines separately in order to be self-crossed for stabilization;
3) Measurement of characteristics: investigating the recombinant individual plants in assortment-related characteristics and determining other characteristics related to the breeding goals after the individual plants have been sorted into groups; collecting sufficient number of seeds from each plant for subsequent planting and characteristic mesurements;
4) Grouping assortment: merging the recombinant individuals or plant lines with the same phenotype into one group according to predetermined characteristics such as plant height;
5) Improving colony varieties: mixing the seeds of corresponding individual plants in the same group in a specific ratio to form a colony variety with a uniform phenotype.

3. The method for breeding colony varieties of crops according to Claim 1 or 2, **characterized in that** said female group has 2-4 original parental lines.

4. The method for breeding colony varieties of crops, according to Claim 3, **characterized in that** each said original parental line of said female group is a homozygous breeding line, a variety or a heterozygous individual plant of the early segregation generations.

5. The method for breeding colony varieties of crops according to Claim 4, **characterized in that** said segregation generations of the female group are the segregation generations of a single cross, double cross or triple cross between their original parental lines.

6. The method for breeding colony varieties of crops according to Claim 1 or 2, **characterized in that** said individual plants in the segregation population of the female group are self-crossed for 1-10 generations.

7. The method for breeding colony varieties of crops according to Claim 1 or 2, **characterized in that** the number of individual plants in the segregation population of the female group or in their self-crossed descendants for hybridization with the male group ranges from 4 to 100,000.

8. The method for breeding colony varieties of crops according to Claim 1 or 2, **characterized in that** the segregation generations of the male group are the segregation generations of a single cross, a double cross or a triple cross between their original parental lines.

9. The method for breeding colony varieties of crops according to Claim 1 or 2,
**characterized in that** the female or male group is a self-pollinating crop, such as rice, peanut, wheat, soybean, and tomato etc; or is a frequently cross-pollinating crop, such as rape, cotton, chili pepper, flowering Chinese cabbage, eggplant, and cabbage mustard etc.

10. The method for breeding colony varieties of crops according to Claim 1 or 2, wherein the grouping assortment of stable population is based on their growth duration, plant height or characteristics of the harvested organs.

11. The use of the method for breeding colony varieties of crops according to Claim 1 or 2, **characterized in that** selecting individual plants or parts of them which accord with the predetermined breeding goals of a colony variety, propagating for several generations to form homozygous breeding lines or multi-genotype varieties with said specific goal.
